# EUROPEAN PATENT APPLICATION

(11) **EP 4 578 379 A1**
(43) Date of publication of application: **02.07.2025**
(21) Application number: 24189178.7
(22) Date of filing: 17.07.2024
(51) Int. Cl.: A61B 5/0205, A61B 5/024, A61B 5/18, A61B 5/291, A61B 5/00, A61B 5/01, A61B 5/11, A61B 5/1171, B60H 1/00, B60J 3/04

(54) **METHOD AND SYSTEM FOR PROVIDING SLEEP CARE**

(30) Priority: 26.12.2023 KR 20230191449; 26.12.2023 KR 20230191450; 26.12.2023 KR 20230191451
(71) Applicant: Hyundai Mobis Co., Ltd., Gangnam-gu Seoul 06141 (KR)
(72) Inventor: PARK, Yeong Hun, 05805 Seoul (KR); PARK, Ki Hee, 13824 Gwacheon-si (KR); LEE, Chang Won, 05532 Seoul (KR)
(74) Representative: Frenkel, Matthias Alexander

(57) **Abstract**

Embodiments of the present disclosure provide a method and system for providing sleep care for an occupant in a vehicle by controlling devices in the vehicle based on sleep information received through an input unit or a sleep state monitored through a sensor part.

## Description

### BACKGROUND OF THE DISCLOSURE

### TECHNICAL FIELD

The present disclosure relates to a method and system for providing sleep care that provides various functions for caring for the sleep of an occupant in a vehicle while the vehicle is moving or stationary.

### BACKGROUND

With the development of autonomous driving technology, other activities besides driving are becoming possible in a vehicle while the vehicle is traveling. In other words, with the advent of the autonomous driving era, occupant convenience can be achieved by allowing occupants to relax in the vehicle. For example, autonomous vehicles allow occupants to sleep while the vehicle is traveling.

However, sleep in a moving vehicle is allowed to last for a relatively short period of time due to various distractions such as vehicle vibrations and external noise. Furthermore, if the occupant enters a deep sleep in the vehicle and wakes up immediately afterward, he or she may feel tired.

Furthermore, there may be multiple occupants in the vehicle. In some cases, not all of occupants may want to sleep. Even if all the occupants want to sleep, the occupants may have different preferences for the in-vehicle sleep environment in terms of a restful sleep.

Furthermore, the occupants may have different destinations, which may result in different travel times within the vehicle. Accordingly, the occupants may want different sleep times.

Therefore, in order to support the restful sleep of occupants in a vehicle, a means for acquiring sleep-related information about occupants, automatically determining whether the occupants are sleeping, and efficiently controlling an in-vehicle device is required.

### SUMMARY

Accordingly, the present disclosure is directed to providing a method and system for providing sleep care. More specifically, an object of the present disclosure is to provide a method and system for providing sleep care that provide various functions for caring for the sleep of an occupant in a vehicle while the vehicle is traveling or stationary.

Another object of the present disclosure is to provide a method and system for providing sleep care which are capable of caring for the sleep of an occupant by controlling a device in a vehicle based on sleep information received through an input unit or a sleep state monitored using a sensor part.

Another object of the present disclosure is to provide a method and system for providing sleep care which are capable of providing a customized sleep environment for each of multiple occupants in a vehicle.

Additional advantages, objects, and features of the disclosure will be set forth in part in the description which follows and in part will become apparent to those having ordinary skill in the art upon examination of the following or may be learned from practice of the disclosure. The objectives and other advantages of the disclosure may be realized and attained by the structure particularly pointed out in the written description and claims hereof as well as the appended drawings.

In one aspect of the present disclosure, a method for providing sleep care for an occupant in a vehicle may include receiving sleep information from the occupant through an input unit or monitoring a sleep state of the occupant through a sensor part, and controlling a lighting device installed in the vehicle based on the received sleep information or the monitored sleep state, wherein the controlling of the lighting device may include switching the lighting device to a sleep mode to care for a sleep of the occupant, and switching the lighting device to a wake-up mode to induce the occupant to wake up.

The sensor part may include a biosensor of a wearable type wearable by the occupant, wherein the biosensor may monitor a vital sign of one or more of brain waves, heart rate, electrocardiogram, or pulse of the occupant.

The biosensor may be configured to determine a feature of the vital sign based on a 1-dimensional convolutional neural network (1D CNN), and classify a sleep state of the occupant based on the determined feature of the vital sign.

The classified sleep state may include one or more of an awake state, a rapid eye movement (REM) sleep state, or a non-REM sleep state.

The received sleep information may include one or more of a travel time, a target sleep time, a preferred light color, or a light brightness.

The controlling of the lighting device further may include controlling the lighting device installed in the vehicle based on sleep information received from each of a plurality of occupants in the vehicle.

The lighting device may include an ambient light, wherein the controlling of the lighting device may further include controlling one or more of a variable sunroof or a variable tinting installed on the vehicle.

The switching to the sleep mode and the switching to the wake-up mode include varying one or more of brightness of the ambient light, transparency of the variable sunroof, or transparency of the variable tinting.

The method may further include changing the vehicle to an autonomous driving mode based on the received sleep information or the monitored sleep state, and outputting information indicative of the occupant's sleep to an outside of the vehicle.

The method may further include controlling a seat or a sound device installed in the vehicle based on the received sleep information or the monitored sleep state, wherein the controlling of the seat or the sound device may include switching the seat or the sound device to the sleep mode to care for the occupant's sleep, and switching the seat or the sound device to the wake-up mode to induce the occupant to wake up.

The received sleep information may include one or more of a travel time, a target sleep time, a preferred seat position, a sound volume, or a sound type.

The controlling of the seat or the sound device may further include controlling the seat or the sound device installed in the vehicle based on sleep information received from each of a plurality of occupants in the vehicle.

The method may further include controlling an air conditioning device installed in the vehicle based on the received sleep information or the monitored sleep state, wherein the controlling of the air conditioning device may include switching the air conditioning device to the sleep mode to care for the occupant's sleep, and switching the air conditioning device to the wake-up mode to induce the occupant to wake up.

The received sleep information may include one or more of a travel time, a target sleep time, a preferred interior temperature, an interior humidity, or an airflow intensity.

The controlling of the air conditioning device may include controlling the air conditioning device installed in the vehicle based on sleep information received from each of a plurality of occupants in the vehicle.

The switching to the sleep mode and the switching to the wake-up mode include varying one or more of a direction, a temperature, or intensity of airflow output from the air conditioning device.

The vehicle may further include a camera configured to capture an image of a change in facial position and temperature of the occupant, wherein the controlling of the air conditioning device may include controlling the air conditioning device based on the captured image of the change in the facial position and temperature.

In another aspect of the present disclosure, a system for providing sleep care for an occupant in a vehicle may include an input unit configured to receive sleep information from the occupant, a sensor part configured to monitor a sleep state of the occupant, and a controller configured to control a lighting device installed in the vehicle based on the sleep information received through the input unit or the sleep state monitored through the sensor part, wherein the controller may be configured to switch the lighting device to a sleep mode to care for a sleep of the occupant and to switch the lighting device to a wake-up mode to induce the occupant to wake up.

The controller may be configured to control a seat or a sound device installed in the vehicle based on the sleep information received through the input unit or the sleep state monitored through the sensor part, switch the seat or the sound device to the sleep mode to care for the occupant's sleep, and switch the seat or the sound device to the wake-up mode to induce the occupant to wake up.

The controller may be configured to control an air conditioning device installed in the vehicle based on the sleep information received through the input unit or the sleep state monitored through the sensor part, switch the air conditioning device to the sleep mode to care for the occupant's sleep, and switch the air conditioning device to the wake-up mode to induce the occupant to wake up.

A method and system for providing sleep care according to the present disclosure may provide various functions for caring for the sleep of an occupant in a vehicle while the vehicle is traveling or stationary.

In addition, sleep of occupants may be taken care of by controlling devices in the vehicle based on sleep information received through an input unit or the sleep state monitored using a sensor part.

Furthermore, a customized sleep environment may be provided for each of occupants in the vehicle.

Further scope of applicability of the present disclosure will become apparent from the following detailed description. Various changes and modifications within the spirit and scope of the present disclosure can be clearly understood by those skilled in the art, and therefore it is to be understood that the detailed description and specific embodiments such as preferred embodiments of the present disclosure are given by way of example only.

### BRIEF DESCRIPTION OF THE DRAWINGS

The accompanying drawings, which are included to provide a further understanding of the invention and are incorporated in and constitute a part of this application, illustrate embodiment(s) of the present disclosure and together with the description serve to explain the principle of the present disclosure. In the drawings:
FIG. 1 is a block diagram of a sleep care providing system according to one embodiment of the present disclosure;
FIG. 2 is a diagram illustrating a wearable type of a biosensor in the sleep care providing system according to one embodiment of the present disclosure;
FIG. 3 is a diagram illustrating how the sleep state of an occupant is monitored using the biosensor of FIG. 2;
FIG. 4 is a diagram illustrating a method for providing sleep care according to one embodiment of the present disclosure;
FIG. 5 is a diagram specifically illustrating a sleep mode and a wake-up mode in the method for providing sleep care according to one embodiment of the present disclosure;
FIG. 6 is a diagram illustrating a method for caring for the sleep of an occupant while a vehicle is traveling in the method for providing sleep care according to one embodiment of the present disclosure;
FIG. 7 is a diagram illustrating another embodiment of controlling a lighting device in the method for providing sleep care according to the present disclosure;
FIG. 8 is a block diagram of a sleep care providing system according to one embodiment of the present disclosure;
FIG. 9 is a diagram illustrating a method for providing sleep care according to one embodiment of the present disclosure;
FIG. 10 is a diagram specifically illustrating a sleep mode and a wake-up mode in the method for providing sleep care according to one embodiment of the present disclosure;
FIG. 11 is a diagram illustrating another embodiment of controlling a seat in the method for providing sleep care according to one embodiment of the present disclosure;
FIG. 12 is a block diagram of a sleep care providing system according to one embodiment of the present disclosure;
FIG. 13 is a diagram illustrating a method for providing sleep care according to one embodiment of the present disclosure; and
FIG. 14 is a diagram specifically illustrating a sleep mode and a wake-up mode in the method for providing sleep care according to one embodiment of the present disclosure.

### DETAILED DESCRIPTION

Description will now be given in detail according to exemplary embodiments disclosed herein, with reference to the accompanying drawings. The same or equivalent components may be provided with the same reference numbers, and description thereof will not be repeated. As used herein, the suffixes "module" and "part" are added or used interchangeably to facilitate preparation of this specification and are not intended to suggest distinct meanings or functions. In describing embodiments disclosed in this specification, relevant well-known technologies may not be described in detail in order not to obscure the subject matter of the embodiments disclosed in this specification. In addition, it should be noted that the accompanying drawings are only for easy understanding of the embodiments disclosed in the present specification, and should not be construed as limiting the technical spirit disclosed in the present specification. As such, the present disclosure should be construed to extend to any alterations, equivalents and substitutes in addition to those which are particularly set out in the accompanying drawings.

Although the terms first, second, etc. may be used herein to describe various elements, these elements should not be limited by these terms. These terms are generally only used to distinguish one element from another.

It will be understood that when an element is referred to as being "connected with" another element, the element can be directly connected with the other element or intervening elements may also be present. In contrast, it will be understood that when an element is referred to as being "directly connected with" another element, there are no intervening elements present.

A singular representation may include a plural representation unless it represents a definitely different meaning from the context.

The terms such as "include" or "have" used herein are intended to indicate that features, numbers, steps, operations, elements, components, or combinations thereof used in the following description exist and it should be thus understood that the possibility of existence or addition of one or more different features, numbers, steps, operations, elements, components, or combinations thereof is not excluded.

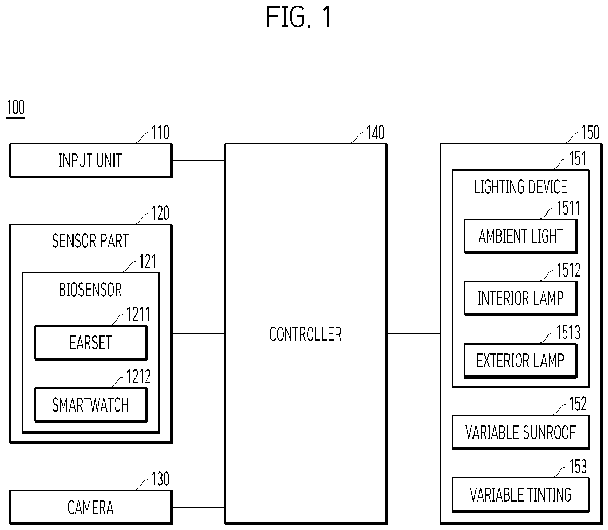

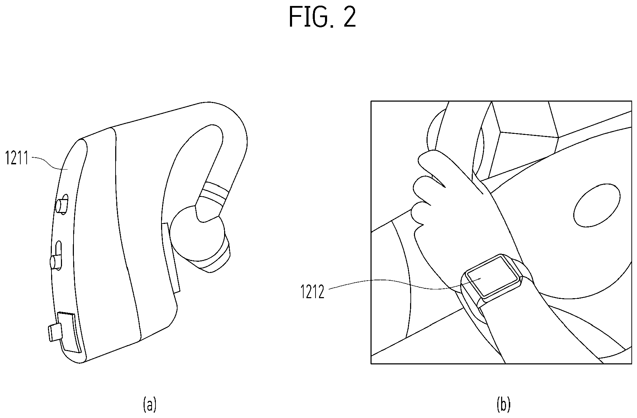

FIG. 1 is a block diagram of a sleep care providing system 100 according to one embodiment of the present disclosure, and FIG. 2 is a diagram illustrating an earset 1211 and a smartwatch 1212, which are wearable types of a biosensor 121 in the sleep care providing system 100 according to one embodiment of the present disclosure.

First, the sleep care providing system 100 according to one embodiment of the present disclosure may include an input unit 110 and a sensor part 120. Here, the input unit 110 may serve to receive sleep information from an occupant of a vehicle. For example, the occupant may input a travel time or a target sleep time through the input unit 110. In addition, the occupant may input a preferred light color for sleeping, and may input a desired light brightness.

The sensor part 120 may be configured to monitor the occupant's sleep state. Here, the sensor part 120 may include a biosensor 121 of a wearable type that may be worn by the occupant. In addition, one or more of the following vital signs of the occupant may be monitored through the biosensor 121: electroencephalogram, heart rate, electrocardiogram, and pulse.

For example, the biosensor 121 may include an earset 1211 configured to measure an electroencephalogram (EEG) signal, as shown in (a) of FIG. 2. The earset 1211 may be mounted around the occupant's ears to measure EEG signals around the occupant's left or right temporal lobe. The biosensor 121 may also include a smartwatch 1212 configured to measure a photoplethysmogram (PPG) signal, as shown in (b) of FIG. 2. The smartwatch 1212 may be worn on the wrist of the occupant and may measure vital signs, such as heart rate, from the occupant's blood flow.

Additionally, the sleep care providing system 100 according to one embodiment of the present disclosure may include a camera 130. The camera 130 may serve to check the facial position of the occupant. Further, the camera 130 may be used to determine the illumination level of the occupant's facial area when controlling the lighting device 151 for care for the occupant's sleep. Furthermore, the position and angle of the seat may be adjusted while checking the occupant's facial position through the camera 130.

Also, the sleep care providing system 100 according to one embodiment of the present disclosure may include a controller 140 and a device 150 including the lighting device 151, the device 150 being installed in a vehicle. The controller 140 may serve to control the lighting device 151 installed in the vehicle based on sleep information received through the input unit 110 or a sleep state monitored through the sensor part 120. Furthermore, the controller 140 may serve to switch the lighting device 151 to a sleep mode to care for the sleep of the occupant, and to switch the lighting device 151 to a wake-up mode to induce the occupant to wake up.

Here, the lighting device 151 may include an ambient light 1511, an interior lamp 1512, and an exterior lamp 1513. Furthermore, the device 150 installed in the vehicle may include a variable sunroof 152 and variable tinting 153. The variable sunroof 152 and variable tinting 153 may function as blackout shades to prevent outside light from disturbing the occupant's sleep. The present disclosure is not limited thereto. In the sleep care providing system 100 according to one embodiment of the present disclosure, the device 150 installed in the vehicle may include blackout curtains to prevent outside light from disturbing the occupant's sleep.

Controlling the lighting device 151, the variable sunroof 152, and the variable tinting 153 by the controller 140 to provide sleep care for the occupants will be described in details later.

It should be noted that the block diagram of the sleep care providing system 100 illustrated in FIG. 1 is merely a block diagram for one embodiment of the present disclosure, and components in the block diagram may be integrated, added or omitted depending on the specifications of the actual implemented sleep care providing system 100.

That is, two or more components may be combined into a single component, or a single component may be subdivided into two or more components, as needed. Furthermore, the functions performed by the respective blocks are intended to illustrate embodiments of the present disclosure, and the specific operations or devices thereof are not intended to limit the scope of the present disclosure.

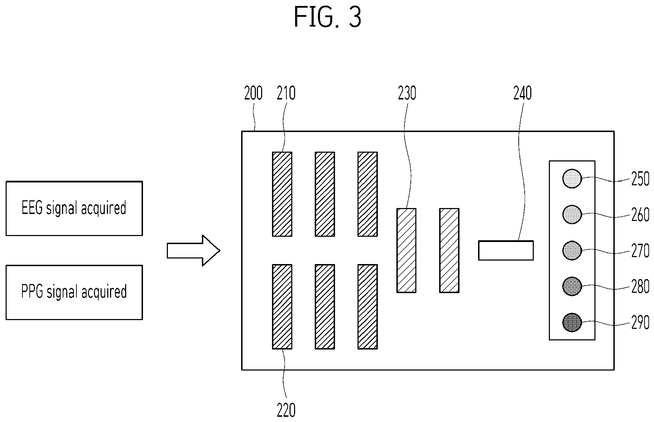

FIG. 3 is a diagram illustrating how the sleep state of an occupant is monitored using the biosensor 121 of FIG. 2.

As described above, the sleep care providing system 100 according to one embodiment of the present disclosure may include the biosensor 121 of a wearable type that may be worn by the occupant,. The biosensor 121 may monitor one or more of the following vital signs of the occupant: EEG, heart rate, electrocardiogram, and pulse.

In particular, the biosensor 121 may determine features of the vital signs based on a 1-dimensional convolutional neural network (1D CNN) technique, and classify the sleep state of the occupant based on the determined features of the vital signs.

In the sleep care providing system 100 according to one embodiment of the present disclosure, the biosensor 121 may include the earset 1211 configured to measure an EEG signal and the smartwatch 1212 configured to measure a PPG signal, as described above with reference to FIG. 2.

In other words, as shown in FIG. 3, the EEG signal of the occupant may be acquired by the earset 1211 and the PPG signal of the occupant may be acquired by the smartwatch 1212. Then, based on the acquired EEG signal and PPG signal as input data, a deep learning model 200 may classify the sleep state of the occupant and manage the sleep of the occupant based on the classification.

The sleep care provision system 100 according to one embodiment of the present disclosure may calculate a feature 210 of the EEG signal and a feature 220 of the PPG signal by applying the 1D CNN. Further, in integrated feature 230 may be calculated by applying the 1D CNN to the feature 210 of the EEG signal and the feature 220 of the PPG signal.

A feature 240 may be extracted from the integrated feature 230 through a fully connected layer, and may be used to classify the sleep state of the occupant based on Softmax. In this way, Softmax may be used to classify the sleep state of the occupant into an awake state 250, REM sleep state 260, and non-REM (NREM) sleep states 270, 280, and 290.

Here, the NREM sleep states 270, 280, and 290 may be categorized into stages based on the depth of the occupant's sleep. For example, the NREM sleep state 270, 280, 290 may be categorized into a state corresponding to a light sleep stage and a state corresponding to a deep sleep stage. The sleep of the occupant may be cared for by distinguishing between the state in which the occupant is in the light sleep stage and the state in which the occupant is in the deep sleep stage.

Therefore, the sleep care provision system 100 according to one embodiment of the present disclosure may classify the sleep state of the occupant step by step through the deep learning model 200 of the 1D CNN described above using the EEG signal and the PPG signal of the occupant as inputs, and care for the sleep of the occupant based on the classification. Hereinafter, a method for managing the sleep of an occupant will be described more specifically.

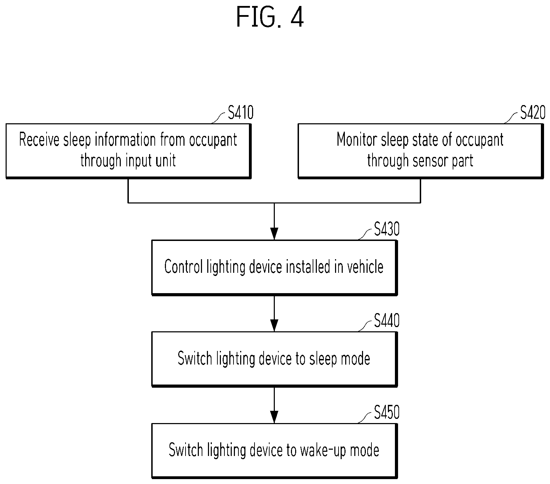

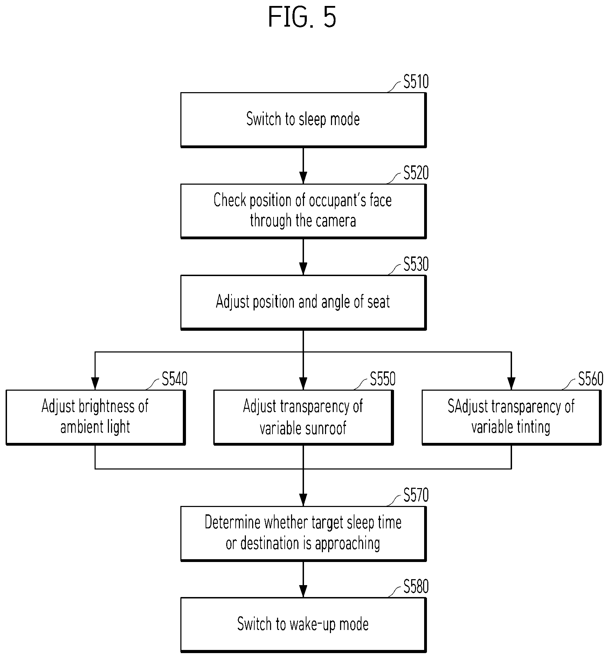

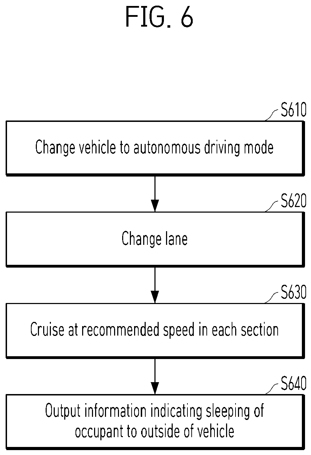

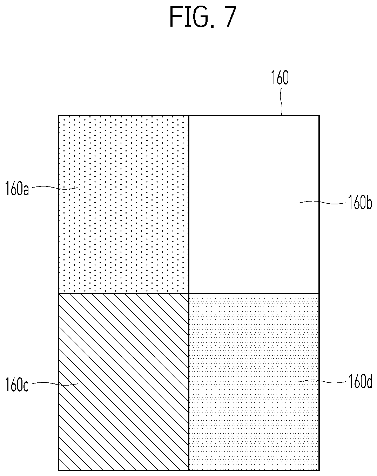

FIG. 4 is a diagram illustrating a method for providing sleep care according to one embodiment of the present disclosure. FIG. 5 is a diagram specifically illustrating a sleep mode and a wake-up mode in a method for providing sleep care according to one embodiment of the present disclosure. FIG. 6 is a diagram illustrating a method for caring for the sleep of an occupant while a vehicle is traveling in the method for providing sleep care according to one embodiment of the present disclosure. FIG. 7 is a diagram illustrating another embodiment of controlling a lighting device in the method for providing sleep care according to the present disclosure.

Hereinafter, a method for providing sleep care according to one embodiment of the present disclosure will be described based on the foregoing description with reference to FIGS. 1 to 3.

Referring to FIG. 4, sleep information may be received from an occupant through the input unit 110 (S410). Here, the sleep information received through the input unit 110 may include one or more of a travel time, a target sleep time, a preferred light color, and a light brightness. This is intended to manage the sleep of the occupant according to the sleep information input by the occupant.

The sleep state of the occupant may be monitored through the sensor part 120 (S420). Here, the sensor part 120 may include the biosensor 121 of a wearable type that may be worn by the occupant, as described above. The biosensor 121 may monitor one or more of the following vital signs of the occupant: EEG, heart rate, electrocardiogram, and pulse.

For example, depending on the travel time required to reach a destination, the occupant may want to take a short sleep lasting 30 minutes or less, or a long sleep lasting an hour or more.

In particular, if the occupant wakes up upon falling into deep sleep, the short sleep may result in a more tired condition, leading to slower cognitive recovery. Therefore, in this case, it is necessary to care for the occupant's sleep such that the occupant does not fall into deep sleep.

Also, when the occupant takes a long sleep, it is necessary to care for the occupant's sleep such that deep sleep is induced. Furthermore, it is necessary to care for the occupant's sleep such that the occupant can wake up from a light sleep state because the occupant may feel more tired if the occupant wakes up after falling into deep sleep as described above.

Therefore, the method for providing sleep care according to one embodiment of the present disclosure may include controlling the lighting device 151 installed in the vehicle based on the sleep information received from the occupant and the monitored sleep state to provide an optimal sleep environment (S430). The controlling of the lighting device 151 may include switching the lighting device 151 to a sleep mode to care for the sleep of an occupant (S440) and switching the lighting device 151 to a wake-up mode to induce the occupant to wake up (S450).

More specifically, referring to FIG. 5, after switching to the sleep mode (S510), the position of the occupant's face may be checked through the camera 130 (S520). Further, the illumination level of the occupant's facial area may be checked through the camera 130 in controlling the lighting device 151 to provide sleep care for the occupant (S530). Furthermore, the position and angle of the seat may be adjusted while checking the position of the occupant's face through the camera 130 (S530).

Further, the method for providing sleep care according to one embodiment of the present disclosure may include adjusting the brightness of the ambient light 1511 (S540), adjusting the transparency of the variable sunroof 152 (S550), or adjusting the transparency of the variable tinting 153 through the controller 140 described above (S560). However, the present disclosure is not limited thereto. The method for providing sleep care according to one embodiment of the present disclosure may include operating blackout curtains, which may be installed in the vehicle, to prevent outside light from disturbing the occupant's sleep.

Then, when it is determined that the target sleep time or the destination is approaching (S570), the system may switch to the wake-up mode (S580).

In one embodiment, when the occupant wants to take a short sleep of 30 minutes or less, the ambient light 1511 and the interior lamp 1512 may be operated with a preferred light color input by the occupant or red light (1900K) for the first five minutes, and the brightness may be varied from 100 lux to 5 lux to induce the occupant to quickly fall into sleep. Then, to prevent the occupant from falling into deep sleep as described above, the ambient light 1511 and the interior lamp 1512 may be continuously operated with a brightness control of 10 lux or less to keep the occupant in a light sleep state. Then, after 30 minutes, the ambient light 1511 and the interior lamp 1512 may be operated with a brightness of 1000 lux or more and white light (4000K) to wake the occupant up.

In another embodiment, when the occupant wants to take a long sleep of one hour or more, the ambient light 1511 and the interior lamp 1512 may be operated with a preferred light color input by the occupant or red light (1900K). Furthermore, the transparency of the variable sunroof 152 and variable tinting 153 may be controlled to gradually change from 100% to 0% to provide a blackout effect in the vehicle. In addition, the ambient light 1511 and the interior lamp 1512 may be controlled to operate at a brightness level of 5 lux or less to keep the occupant in a deep sleep state. Furthermore, the occupant may set the ambient light 1511 and interior lamp 1512 to be powered off after a certain period of time. The setting may be included in the sleep information received through the input unit 110 described above.

Here, it is necessary to it is necessary to care for the occupant's sleep such that the occupant can wake up from a light sleep state because the occupant may feel more tired if the occupant wakes up after falling into deep sleep as described above. To this end, when it is determined that a target sleep time or a destination is approaching, the ambient light 1511 and the interior lamp 1512 may be controlled to gradually brighten over a period of time. Similarly, the transparency of the variable sunroof 152 and variable tinting 153 may be controlled to gradually change.

Thereafter, upon reaching the target sleep time or arriving at the destination, the ambient light 1511 and interior lamp 1512 may be operated with a gradual change in brightness from 0 lux to 300 lux over a predetermined period of time (e.g., 3 minutes), followed by a brightness of 1000 lux or greater. Additionally, the variable sunroof 152 and the variable tinting 153 may be operated to have 100% transparency. This may induce the occupant to wake up.

Furthermore, the method for providing sleep care according to one embodiment of the present disclosure may provide sleep care for an occupant not only when the vehicle is stationary, but also when the vehicle is traveling. In this case, it is necessary for the vehicle to be controlled such that the safety of the occupant is ensured while the occupant's sleep is cared for as described above.

Thus, referring to FIG. 6, a method for providing sleep care according to one embodiment of the present disclosure may include changing the vehicle to an autonomous driving mode (S610) and changing a lane (S620). Furthermore, the vehicle may be caused to cruise at a recommended speed in each section (S630). For example, on a three-lane road with a speed limit of 60 km/h, the vehicle may be caused to change the lane to the outermost lane, the third lane, and travel at a cruising speed that does not exceed the speed limit.

Furthermore, information indicating that the occupant is sleeping may be output to the outside of the vehicle (S640). In one embodiment, the exterior lamp 1513 may output information indicating that the occupant is sleeping. For example, an image or text that may indicate the occupant's sleep may be output on the road surface around the vehicle through the exterior lamp 1513. The present disclosure is not limited thereto. An image or text that may indicate the occupant's sleep may be output on a window or external display installed on the vehicle.

In addition, the method for providing sleep care according to the present disclosure may provide a customized sleep environment for each of multiple occupants in the vehicle. This may be accomplished by receiving sleep information from each of the occupants in the vehicle through the input unit 110 described above and controlling the lighting device 151 installed in the vehicle based on the sleep information.

Referring to FIG. 7, for example, when four occupants are in the vehicle, the interior area 160 of the vehicle may be divided into four zones 160a, 160b, 160c, and 160d based on the seating positions of the occupants. And each occupant may input the sleep information described above, such as a preferred light color or light brightness. Based on the input information, the color or brightness of the ambient light 1511 may be controlled, thereby implementing sleep care for multiple occupants.

The present disclosure is not limited thereto. The interior area 160 of the vehicle may be equipped with a plurality of sun caps or masks. Furthermore, the vehicle may be equipped with a member for blocking external light that may be directed at the faces of the multiple occupants.

In some cases, there may be occupants who want to sleep and occupants who do not want to sleep. In this case, the ambient light 1511 may be operated only in the zones where occupants who want to sleep are seated. Additionally, outside light may be blocked by varying the transparency of the variable sunroof 152 or variable tinting 153 only in the zones where occupants who want to sleep are seated.

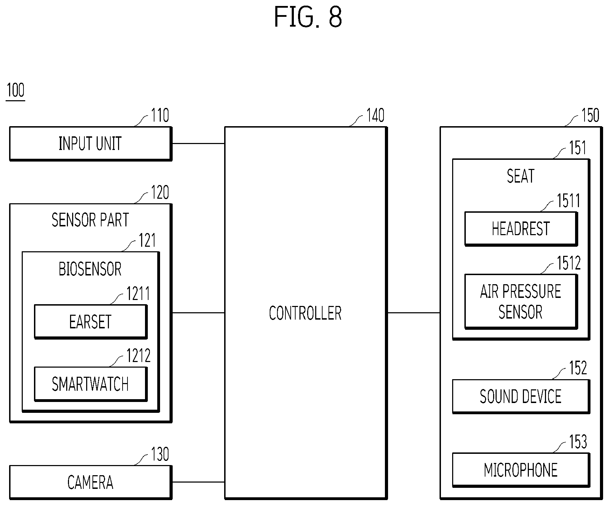

FIG. 8 is a block diagram of a sleep care providing system 100 according to one embodiment of the present disclosure.

First, the sleep care providing system 100 according to one embodiment of the present disclosure may include an input unit 110 and a sensor part 120. Here, the input unit 110 may serve to receive sleep information from an occupant of a vehicle. For example, the occupant may input a travel time or a target sleep time through the input unit 110. In addition, the occupant may input the position of a preferred seat 151 for sleeping, and may input a volume and type of desired sound.

The sensor part 120 may be configured to monitor the occupant's sleep state. Here, the sensor part 120 may include a biosensor 121 of a wearable type that may be worn by the occupant. In addition, one or more of the following vital signs of the occupant may be monitored through the biosensor 121: EEG, heart rate, electrocardiogram, and pulse.

For example, the biosensor 121 may include an earset 1211 configured to measure an EEG signal, as shown in (a) of FIG. 2. The earset 1211 may be mounted around the occupant's ears to measure EEG signals around the occupant's left or right temporal lobe. The biosensor 121 may also include a smartwatch 1212 configured to measure a photoplethysmogram (PPG) signal, as shown in (b) of FIG. 2. The smartwatch 1212 may be worn on the wrist of the occupant and may measure vital signs, such as heart rate, from the occupant's blood flow.

Additionally, the sleep care providing system 100 according to one embodiment of the present disclosure may include a camera 130. The camera 130 may serve to check the facial position of the occupant. Further, the camera 130 may be used to determine the posture of the occupant. For example, the camera 130 may be used to track the joints of the occupant to determine the positions of the occupant's head, neck, waist, and legs. Accordingly, the position and angle of the seat 151 may be adjusted based on the determination of the facial position and posture of the occupant based on the camera 130.

Also, the sleep care providing system 100 according to one embodiment of the present disclosure may include a controller 140 and a device 150 including the seat 151 and a sound device 152, the device 150 being installed in the vehicle. The controller 140 may serve to control the seat 151 or sound device 152 installed in the vehicle based on the sleep information received through the input unit 110 or the sleep state monitored through the sensor part 120. Further, the controller 140 may serve to switch the seat 151 or sound device 152 to the sleep mode to care for the sleep of the occupant, and to switch the seat 151 or sound device 152 to induce the occupant to wake up.

Here, the seat 151 may include a headrest 1511 and an air pressure sensor 1512. In one embodiment, the air pressure sensor 1512 may be used to change the shape of the headrest 1511 to adapt to the occupant's facial position. The present disclosure is not limited thereto. The sleep care providing system 100 according to one embodiment of the present disclosure may change the position and shape of the seat 151 to adapt to the occupant's posture, using the camera 130 and the air pressure sensor 1512.

The sound device 152 may serve to care for the occupant's sleep by inducing a specific brain wave of the occupant by outputting an appropriate volume of sound or by playing the sound source output from the left and right speakers differently by the frequency (Hz) of the specific brain wave. Also, the microphone 153 may receive sound input from the occupant. For example, the sound of the occupant snoring during sleep may be input, and the seat 151 may be controlled based on the input sound.

Details of controlling the seat 151 or the sound device 152 through the controller 140 to care for the sleep of the occupant will be described later.

It should be noted that the block diagram of the sleep care providing system 100 illustrated in FIG. 8 is merely a block diagram for one embodiment of the present disclosure, and components in the block diagram may be integrated, added or omitted depending on the specifications of the actual implemented sleep care providing system 100.

That is, two or more components may be combined into a single component, or a single component may be subdivided into two or more components, as needed. Furthermore, the functions performed by the respective blocks are intended to illustrate embodiments of the present disclosure, and the specific operations or devices thereof are not intended to limit the scope of the present disclosure.

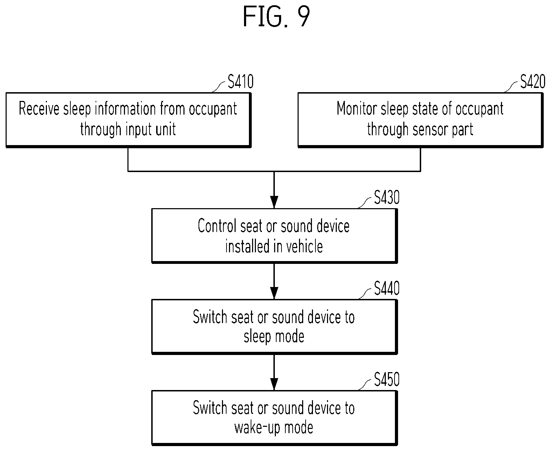

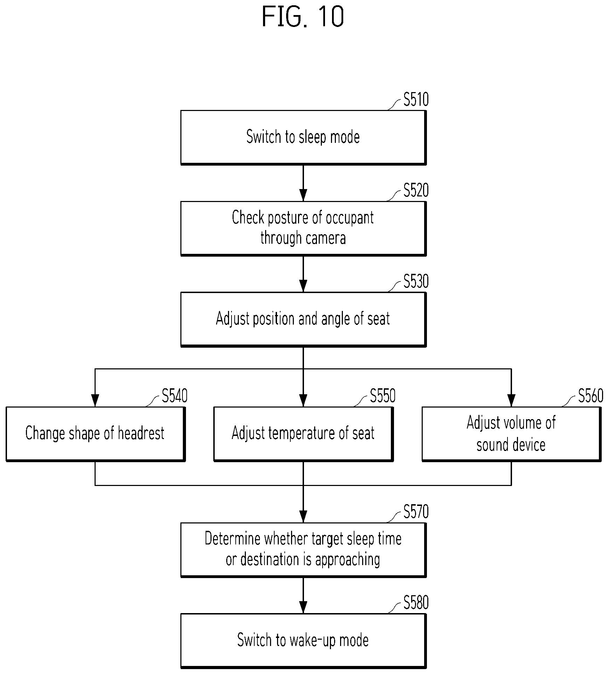

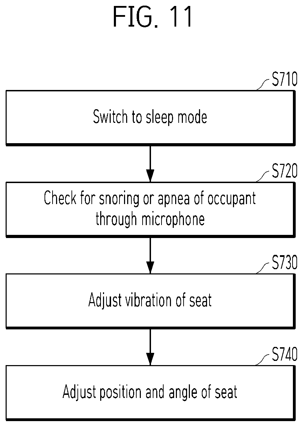

FIG. 9 is a diagram illustrating a method for providing sleep care according to one embodiment of the present disclosure. FIG. 10 is a diagram specifically illustrating a sleep mode and a wake-up mode in the method for providing sleep care according to one embodiment of the present disclosure. FIG. 11 is a diagram illustrating another embodiment of controlling a seat in the method for providing sleep care according to the present disclosure.

Hereinafter, a method for providing sleep care according to one embodiment of the present disclosure will be described based on the foregoing description with reference to FIGS. 2, 3, and 8.

Referring to FIG. 9, sleep information may be received from an occupant through the input unit 110 (S410). Here, the sleep information received through the input unit 110 may include one or more of a travel time, a target sleep time, a position of the preferred seat 151, a sound volume, and a sound type. This is intended to manage the sleep of the occupant according to the sleep information input by the occupant.

The sleep state of the occupant may be monitored through the sensor part 120 (S420). Here, the sensor part 120 may include the biosensor 121 of a wearable type that may be worn by the occupant, as described above. The biosensor 121 may monitor one or more of the following vital signs of the occupant: EEG, heart rate, electrocardiogram, and pulse.

For example, depending on the travel time required to reach a destination, the occupant may want to take a short sleep lasting 30 minutes or less, or a long sleep lasting an hour or more.

In particular, if the occupant wakes up upon falling into deep sleep, the short sleep may cause the occupant to feel more tired, resulting in slower cognitive recovery. Therefore, in this case, it is necessary to care for the occupant's sleep such that the occupant does not fall into deep sleep.

Also, when the occupant takes a long sleep, it is necessary to care for the occupant's sleep such that deep sleep is induced. Furthermore, it is necessary to care for the occupant's sleep such that the occupant can wake up from a light sleep state because the occupant may feel more tired if the occupant wakes up after falling into deep sleep as described above.

Therefore, the method for providing sleep care according to one embodiment of the present disclosure may control the seat 151 or the sound device 152 installed in the vehicle based on the sleep information received from the occupant and the monitored sleep state to provide an optimal sleep environment (S430). The controlling of the seat 151 or sound device 152 may include switching the seat 151 or sound device 152 to a sleep mode to care for the occupant's sleep (S440) and switching the seat 151 or sound device 152 to a wake-up mode to induce the occupant to wake up (S450).

More specifically, referring to FIG. 10, after switching to the sleep mode (S510), the occupant's posture may be checked through the camera 130 (S520), and the position and angle of the seat 151 may be adjusted based thereon (S530).

Further, the method for providing sleep care according to one embodiment of the present disclosure may include changing the shape of the headrest 1511 (S540), adjusting the temperature of the seat 151 (S550), and adjusting the volume or type of the sound device 152 (S560) through the controller 140 described above. Here, the shape of the headrest 1511 may be changed according to the position of the occupant's face using the air pressure sensor 1512 described above, taking into account the occupant's posture. The seat 151 may vibrate from side to side to care for the occupant's sleep.

Then, when it is determined that the target sleep time or the destination is approaching (S570), the system may switch to the wake-up mode (S580).

In one embodiment, when the occupant wants to take a short sleep of 30 minutes or less, the volume may be adjusted to 40 to 50 dB for the first 5 minutes to induce the occupant to quickly fall into sleep. Then, to prevent the occupant from falling into deep sleep as described above, the sound device 152 may be continuously operated to output left and right sounds with a difference of 4 to 7 Hz, corresponding to theta waves to keep the occupant in the light sleep state. After 30 minutes, the volume may be adjusted to 50 dB or more to wake the occupant up.

In another embodiment, when the occupant wants to take a long sleep of one hour or more, the sound whose volume is increased to about 40 dB may be output. The sound device 152 may then be operated to continuously output left and right sounds with a difference of about 2 to 4 Hz, which corresponds to delta waves, to keep the occupant in a deep sleep state. Furthermore, the occupant may set the sound device 152 to be powered off after a certain period of time. The setting may be included in the sleep information received through the input unit 110 described above.

Here, it is necessary to it is necessary to care for the occupant's sleep such that the occupant can wake up from a light sleep state because the occupant may feel more tired if the occupant wakes up after falling into deep sleep as described above. To this end, when it is determined that a target sleep time or a destination is approaching, the sound output from the sound device 152 may be controlled to gradually increase in volume.

Thereafter, upon reaching the target sleep time or arriving at the destination, the sound device 152 may be operated to output a sound with a volume of 50 dB or more to wake the occupant up. Furthermore, the seat 151 may be controlled to change position and angle of the seat 151 to induce the occupant to wake up.

Further, the method for providing sleep care according to one embodiment of the present disclosure may include adjusting the temperature of the seat 151 to care for the sleep of the occupant. The temperature may be set to a temperature of the seat 151 that the occupant prefers when sleeping, based on the sleep information about the occupant input through the input unit 110.

Furthermore, the method for providing sleep care according to one embodiment of the present disclosure may provide sleep care for an occupant not only when the vehicle is stationary, but also when the vehicle is traveling. In this case, it is necessary for the vehicle to be controlled such that the safety of the occupant is ensured while the occupant's sleep is cared for as described above.

Thus, referring to FIG. 6, a method for providing sleep care according to one embodiment of the present disclosure may include changing the vehicle to an autonomous driving mode (S610) and changing a lane (S620). Furthermore, the vehicle may be caused to cruise at a recommended speed in each section (S630). For example, on a three-lane road with a speed limit of 60 km/h, the vehicle may be caused to change the lane to the outermost lane, the third lane, and travel at a cruising speed that does not exceed the speed limit.

Furthermore, information indicating that the occupant is sleeping may be output to the outside of the vehicle (S640). In one embodiment, the exterior lamp may output information indicating that the occupant is sleeping. For example, an image or text that may indicate the occupant's sleep may be output on the road surface around the vehicle through the exterior lamp. The present disclosure is not limited thereto. An image or text that may indicate the occupant's sleep may be output on a window or external display installed on the vehicle.

Also, referring to FIG. 11, the method for providing sleep care according to one embodiment of the present disclosure may include switching to a sleep mode (S710), and then check for snoring or apnea of the occupant through the microphone 153 (S720). The method may also include adjusting the vibration of the seat 151 (S730), and adjusting the position and angle of the seat 151 (S740). Thereby, the occupant may be prevented from snoring or be caused to wake up when apnea is detected.

In addition, the method for providing sleep care according to the present disclosure may provide a customized sleep environment for each of multiple occupants in the vehicle. This may be accomplished by receiving sleep information from each of the occupants in the vehicle through the input unit 110 described above and controlling the seat 151 or sound device 152 installed in the vehicle based on the sleep information.

In other words, multiple seats 151 may be individually controlled based on the seating positions of multiple occupants. Further, the sound device 152 may be controlled by dividing the interior area of the vehicle based on the seating positions of the multiple occupants in the vehicle. In this case, the sound device 152 may utilize noise cancellation to prevent sleep disturbance due to overlapping sounds between the multiple occupants. The present disclosure is not limited thereto. As described above with reference to FIG. 11, when snoring of some of the multiple occupants is identified through the microphone 153, the seats 151 occupied by such occupants may be controlled so as not to disturb the sleep of the other occupants.

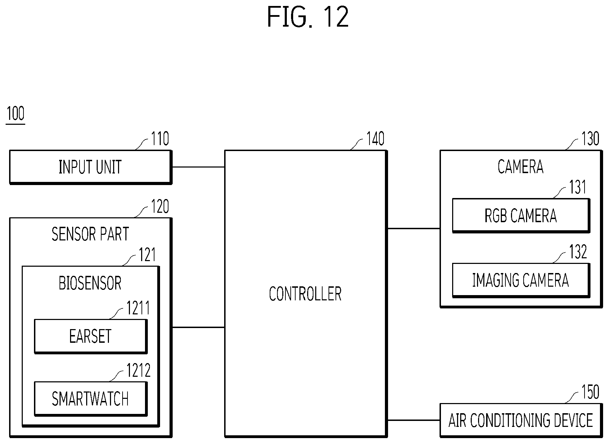

FIG. 12 is a block diagram of a sleep care providing system according to one embodiment of the present disclosure.

First, the sleep care providing system 100 according to one embodiment of the present disclosure may include an input unit 110 and a sensor part 120. Here, the input unit 110 may serve to receive sleep information from an occupant of a vehicle. For example, the occupant may input a travel time or a target sleep time through the input unit 110. In addition, for sleeping, the occupant may input a preferred temperature or airflow intensity inside the vehicle, and input a desired humidity inside the vehicle.

The sensor part 120 may be configured to monitor the occupant's sleep state. Here, the sensor part 120 may include a biosensor 121 of a wearable type that may be worn by the occupant. In addition, one or more of the following vital signs of the occupant may be monitored through the biosensor 121: EEG, heart rate, electrocardiogram, pulse, heart portion temperature, and skin temperature. Here, the skin temperature may include a temperature measured on the occupant's wrist.

For example, the biosensor 121 may include an earset 1211 for measuring EEG signal, as shown in (a) of FIG. 2. The earset 1211 may be mounted around the occupant's ears to measure EEG signals around the occupant's left or right temporal lobe. The earset 1211 may also measure the heart portion temperature of the occupant using a temperature measurement member that may be positioned in the occupant's ear when the earset 1211 are worn. The biosensor 121 may also include a smartwatch 1212 configured to measure a PPG signal, as shown in (b) of FIG. 2. The smartwatch 1212 may be worn on the wrist of the occupant, and may measure vital signs, such as heart rate, from the occupant's blood flow, and measure the temperature of the occupant's skin (wrist) as described above.

Additionally, the sleep care providing system 100 according to one embodiment of the present disclosure may include a camera 130. The camera 130 may serve to check the facial position of the occupant. Further, the camera 130 may include an RGB camera 131 and a thermal imaging camera 132.

In one embodiment, the distance from the dashboard of the vehicle to the occupant's face may be calculated based on the image captured by the RGB camera 131. Additionally, the RGB camera 131 and thermal imaging camera 132 may be used to capture images of the occupant's facial area to measure thermal changes on the occupant's face and skin (facial) temperature. Based on this measurement, the optimal airflow may be directed to the occupant's face to care for the occupant's sleep.

The present disclosure is not limited thereto. The sleep care providing system 100 according to one embodiment of the present disclosure may measure the sound of the airflow through a microphone located in the headrest of a seat installed in the vehicle. Based on the measured sound of the airflow, the airflow intensity may be calculated to adjust the intensity of the airflow directed at the occupant's facial area to provide sleep care for the occupant.

Furthermore, the sleep care provision system 100 according to one embodiment of the present disclosure may check the position of the occupant's face through the camera 130 to adjust the position and angle of the seat installed in the vehicle.

The sleep care provision system 100 according to one embodiment of the present disclosure may include a controller 140 and an air conditioning device 150. The controller 140 may serve to control the air conditioning device 150 installed in the vehicle based on the sleep information received through the input unit 110 or the sleep state monitored through the sensor part 120. In addition, the controller 140 may serve to switch the air conditioning device 150 to a sleep mode to care for the sleep of an occupant, and to switch the air conditioning device 150 to a wake-up mode to wake the occupant up.

Details of controlling the air conditioning device 150 through the controller 140 to care for the sleep of the occupant will be described later.

It should be noted that the block diagram of the sleep care providing system 100 illustrated in FIG. 12 is merely a block diagram for one embodiment of the present disclosure, and components in the block diagram may be integrated, added or omitted depending on the specifications of the actual implemented sleep care providing system 100.

That is, two or more components may be combined into a single component, or a single component may be subdivided into two or more components, as needed. Furthermore, the functions performed by the respective blocks are intended to illustrate embodiments of the present disclosure, and the specific operations or devices thereof are not intended to limit the scope of the present disclosure.

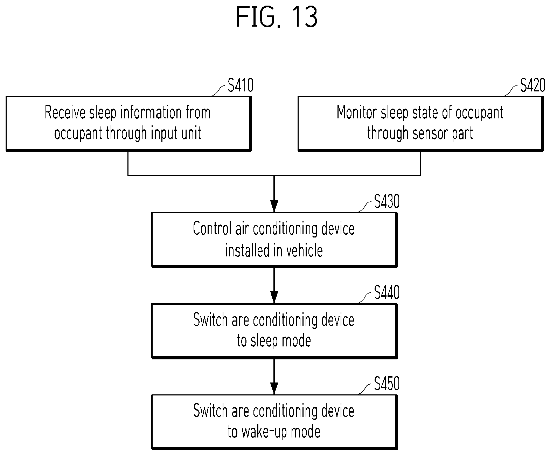

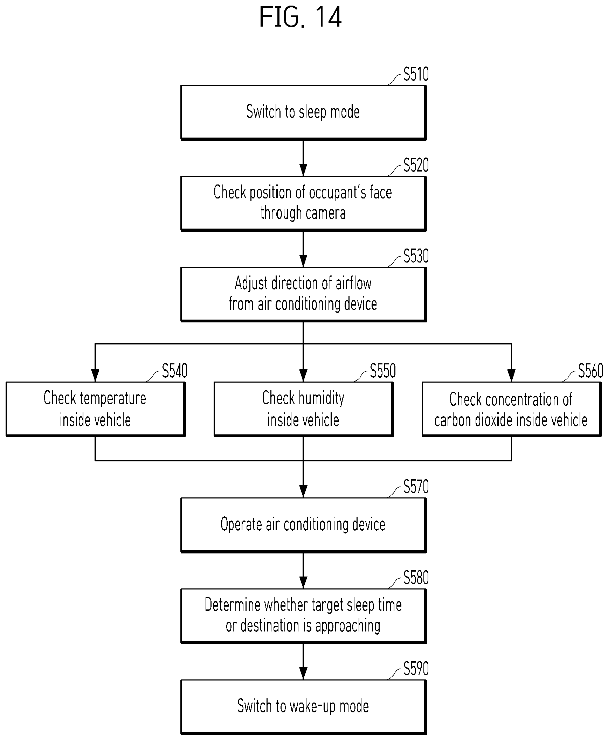

FIG. 13 is a diagram illustrating a method for providing sleep care according to one embodiment of the present disclosure. FIG. 14 is a diagram specifically illustrating a sleep mode and a wake-up mode in the method for providing sleep care according to one embodiment of the present disclosure.

Hereinafter, a method for providing sleep care according to one embodiment of the present disclosure will be described based on the foregoing description with reference to FIGS. 2, 3, and 12.

Referring to FIG. 13, sleep information may be received from an occupant through the input unit 110 (S410). Here, the sleep information received through the input unit 110 may include one or more of a travel time, a target sleep time, a preferred internal temperature, an internal humidity, and an airflow intensity. This is intended to manage the sleep of the occupant according to the sleep information input by the occupant.

The sleep state of the occupant may be monitored through the sensor part 120 (S420). Here, the sensor part 120 may include the biosensor 121 of a wearable type that may be worn by the occupant, as described above. The biosensor 121 may monitor one or more of the following vital signs of the occupant: EEG, heart rate, electrocardiogram, pulse, heart portion temperature, and skin temperature.

For example, depending on the travel time required to reach a destination, the occupant may want to take a short sleep lasting 30 minutes or less, or a long sleep lasting an hour or more.

In particular, if the occupant wakes up upon falling into deep sleep, the short sleep may result in a more tired condition, leading to slower cognitive recovery. Therefore, in this case, it is necessary to care for the occupant's sleep such that the occupant does not fall into deep sleep.

Also, when the occupant takes a long sleep, it is necessary to care for the occupant's sleep such that deep sleep is induced. Furthermore, it is necessary to care for the occupant's sleep such that the occupant can wake up from a light sleep state because the occupant may feel more tired if the occupant wakes up after falling into deep sleep as described above.

Therefore, the method for providing sleep care according to one embodiment of the present disclosure may include controlling the air conditioning device 150 installed in the vehicle based on the sleep information received from the occupant and the monitored sleep state to provide an optimal sleep environment (S430). The controlling of the air conditioning device 150 may include switching the air conditioning device 150 to a sleep mode to care for the sleep of an occupant (S440) and switching the air conditioning device 150 to a wake-up mode to induce the occupant to wake up (S450).

More specifically, referring to FIG. 5, after switching to the sleep mode (S510), the position of the occupant's face may be checked through the camera 130 (S520). Further, while the position of the occupant's face may be checked through the camera 130, the direction of airflow from the air conditioning device 150 may be adjusted to be delivered to the occupant's face (S530). In other words, the angle of the air conditioning device 150 may be adjusted to allow the airflow to be delivered to the occupant's face.

In addition, the method for providing sleep care according to one embodiment of the present disclosure may include checking the temperature inside the vehicle (S540), checking the humidity inside the vehicle (S550), or checking the concentration of carbon dioxide inside the vehicle (S560). Based on the checking, the air conditioning device 150 may be operated (S570) to care for the sleep of the occupant.

In one embodiment, the air conditioner or heater of the air conditioning device 150 may be operated to maintain the temperature inside the vehicle at 24°C to 26°C. For example, the heater may be operated when the temperature inside the vehicle is lower than 24°C, and the air conditioner may be operated when the temperature inside the vehicle is higher than 27°C.

Further, the air conditioner of the air conditioning device 150 may be operated to maintain a humidity of 40% to 60% inside the vehicle. Furthermore, the air conditioning device 150 may be operated such that the concentration of carbon dioxide inside the vehicle is maintained at 900 ppm or less, and outside air may be introduced into the vehicle. The present disclosure is not limited thereto. In the method for providing sleep care according to one embodiment of the present disclosure, the air conditioning device 150 may be operated to cause air to flow towards the occupant's face at about 0.8 m/s.

In addition, the air conditioning device 150 may be controlled to ensure that the occupant's facial area is maintained at an appropriate temperature by continuously measuring thermal changes in the occupant's facial area through the RGB camera 131 and the thermal imaging camera 132 as described above.

In one embodiment, when the temperature of the occupant's facial area has not reached an appropriate temperature, the air conditioning device 150 may be controlled to adjust the airflow intensity and operate the air conditioner or heater to reach the appropriate temperature.

Additionally, the climate control 150 may be controlled to care for the occupant's sleep by monitoring the occupant's heart portion temperature through the earset 1211 worn by the occupant and/or monitoring the temperature of the occupant's skin (wrist) through the smartwatch 1212 worn by the occupant.

Then, when it is determined that the target sleep time or the destination is approaching (S570), the system may switch to the wake-up mode (S580).

In one embodiment, when the occupant wants to take a short sleep of 30 minutes or less, the air conditioner or heater may be operated strongly for the first five minutes to quickly bring the temperature inside the vehicle to a preferred temperature for the occupant to induce the occupant to fall into sleep quickly. Then, to prevent the occupant from falling into deep sleep as described above, the air conditioning device 150 may be operated to maintain the occupant in a light sleep state with the air conditioner or heater operating continuously thereafter. After 30 minutes, the air conditioning device 150 may be operated to provide warm air to the occupant's facial area to wake the occupant up.

In another embodiment, when the occupant wants to take a long sleep of one hour or more, the intensity of the air conditioner or heater may be gradually increased such that the temperature inside the vehicle reaches the temperature preferred by the occupant. Then, the operation of the air conditioner or heater may be controlled to maintain the occupant in a deep sleep state. Furthermore, the occupant may set the air conditioning device 150 to be powered off after a certain period of time. The setting may be included in the sleep information received through the input unit 110 described above.

Here, it is necessary to it is necessary to care for the occupant's sleep such that the occupant can wake up from a light sleep state because the occupant may feel more tired if the occupant wakes up after falling into deep sleep as described above. To this end, when it is determined that a target sleep time or a destination is approaching, the air conditioning device 150 may be operated to gradually change the intensity of the air conditioner or heater.

Thereafter, upon reaching the target sleep time or arriving at the destination, the air conditioning device 150 may be operated to provide warm air to the occupant's facial area to wake the occupant up.

Furthermore, the method for providing sleep care according to one embodiment of the present disclosure may provide sleep care for an occupant not only when the vehicle is stationary, but also when the vehicle is traveling. In this case, it is necessary for the vehicle to be controlled such that the safety of the occupant is ensured while the occupant's sleep is cared for as described above.

Thus, referring to FIG. 6, a method for providing sleep care according to one embodiment of the present disclosure may include changing the vehicle to an autonomous driving mode (S610) and changing a lane (S620). Furthermore, the vehicle may be caused to cruise at a recommended speed in each section (S630). For example, on a three-lane road with a speed limit of 60 km/h, the vehicle may be caused to change the lane to the outermost lane, the third lane, and travel at a cruising speed that does not exceed the speed limit.

Furthermore, information indicating that the occupant is sleeping may be output to the outside of the vehicle (S640). In one embodiment, the exterior lamp may output information indicating that the occupant is sleeping. For example, an image or text that may indicate the occupant's sleep may be output on the road surface around the vehicle through the exterior lamp. The present disclosure is not limited thereto. An image or text that may indicate the occupant's sleep may be output on a window or external display installed on the vehicle.

In addition, the method for providing sleep care according to the present disclosure may provide a customized sleep environment for each of multiple occupants in the vehicle. This may be accomplished by receiving sleep information from each of the occupants in the vehicle through the input unit 110 described above and controlling the air conditioning device 150 installed in the vehicle based on the sleep information.

In other words, the air conditioning device 150 may be controlled by dividing the interior area of the vehicle based on the seating positions of the multiple occupants in the vehicle. Further, the air conditioning device 150 may be controlled to monitor changes in temperature of each of the occupants through the camera 130 to care for the sleep of the occupants. The present disclosure is not limited thereto. The air conditioning device 150 may be controlled to check the temperature, humidity, and carbon dioxide concentration inside the vehicle to care for the sleep of multiple occupants as described above.

In summary, the method and system for providing sleep care according to the present disclosure may provide a variety of functions for caring for the sleep of occupants while the vehicle is traveling or stationary. In addition, the sleep care may be provided by controlling the devices in the vehicle based on sleep information received through an input unit or a sleep state monitored through a sensor part. Furthermore, a customized sleep environment may be provided for each of a plurality of occupants in the vehicle.

The above detailed description is to be construed in all aspects as illustrative and not restrictive. The scope of the present disclosure should be determined by reasonable interpretation of the appended claims and all changes coming within the equivalency range of the present disclosure are intended to be embraced in the scope of the present disclosure.

## Claims

1. A method for providing sleep care for an occupant in a vehicle, the method comprising:
receiving, by a controller, sleep information from the occupant through an input unit operatively connected to the controller or monitoring a sleep state of the occupant through a sensor part operatively connected to the controller; and
controlling, by the controller, a lighting device operatively connected to the controller and installed in the vehicle, based on the received sleep information or the monitored sleep state,
wherein the controlling of the lighting device comprises:
switching the lighting device to a sleep mode to care for a sleep of the occupant, or
switching the lighting device to a wake-up mode to induce the occupant to wake up.

2. The method of claim 1, wherein the sensor part comprises:
a biosensor wearable by the occupant,
wherein the biosensor monitors a vital sign of one or more of brain waves, heart rate, electrocardiogram, or pulse of the occupant.

3. The method of claim 2, wherein the biosensor is configured to:
determine a feature of the vital sign based on a 1-dimensional convolutional neural network (1D CNN); and
classify the sleep state of the occupant based on the determined feature of the vital sign.

4. The method of claim 3, wherein the classified sleep state comprises one or more of an awake state, a rapid eye movement (REM) sleep state, or a non-REM sleep state.

5. The method of any one of claims 1 to 4, wherein the received sleep information comprises one or more of a travel time, a target sleep time, a preferred light color, or a light brightness,
wherein the controlling of the lighting device further comprises:
controlling the lighting device installed in the vehicle based on sleep information received from each of a plurality of occupants in the vehicle.

6. The method of any one of claims 1 to 5, wherein the lighting device comprises an ambient light,
wherein the controlling of the lighting device further comprises:
controlling one or more of a variable sunroof or a variable tinting installed on the vehicle.

7. The method of claim 6, wherein the switching to the sleep mode and the switching to the wake-up mode comprise:
varying one or more of brightness of the ambient light, transparency of the variable sunroof, or transparency of the variable tinting.

8. The method of any one of claims 1 to 7, further comprising:
changing, by the controller, the vehicle to an autonomous driving mode based on the received sleep information or the monitored sleep state; and
outputting, by the controller, information indicative of the occupant's sleep to an outside of the vehicle.

9. The method of any one of claims 1 to 8, further comprising:
controlling, by the controller, a seat or a sound device installed in the vehicle, based on the received sleep information or the monitored sleep state,
wherein the controlling of the seat or the sound device comprises:
switching the seat or the sound device to the sleep mode to care for the occupant's sleep, or
switching the seat or the sound device to the wake-up mode to induce the occupant to wake up.

10. The method of claim 9, wherein the received sleep information comprises one or more of a travel time, a target sleep time, a preferred seat position, a sound volume, or a sound type,
wherein the controlling of the seat or the sound device further comprises:
controlling the seat or the sound device installed in the vehicle based on sleep information received from each of a plurality of occupants in the vehicle.

11. The method of any one of claims 1 to 10, further comprising:
controlling, by the controller, an air conditioning device installed in the vehicle, based on the received sleep information or the monitored sleep state,
wherein the controlling of the air conditioning device comprises:
switching the air conditioning device to the sleep mode to care for the occupant's sleep, or
switching the air conditioning device to the wake-up mode to induce the occupant to wake up.

12. The method of claim 11, wherein the received sleep information comprises one or more of a travel time, a target sleep time, a preferred interior temperature, an interior humidity, or an airflow intensity,
wherein the controlling of the air conditioning device comprises:
controlling the air conditioning device installed in the vehicle based on sleep information received from each of a plurality of occupants in the vehicle.

13. The method of claim 11 or 12, wherein the switching to the sleep mode and the switching to the wake-up mode comprise:
varying one or more of a direction, a temperature, or intensity of airflow output from the air conditioning device.

14. The method of claim 13, wherein the vehicle further comprises a camera configured to capture an image of a change in facial position and temperature of the occupant,
wherein the controlling of the air conditioning device comprises:
controlling the air conditioning device based on the captured image of the change in the facial position and temperature.

15. A system for providing sleep care for an occupant in a vehicle, the system comprising:
an input unit configured to receive sleep information from the occupant;
a sensor part configured to monitor a sleep state of the occupant; and
a controller operatively connected to the input unit and the sensor part and configured to control a lighting device operatively connected to the controller and installed in the vehicle, based on the sleep information received through the input unit or the sleep state monitored through the sensor part,
wherein the controller is configured to:
switch the lighting device to a sleep mode to care for a sleep of the occupant, or
switch the lighting device to a wake-up mode to induce the occupant to wake up.
